# EUROPÄISCHE PATENTSCHRIFT

(11) **EP 1 150 620 B1**
(45) Veröffentlichungstag und Bekanntmachung des Hinweises auf die Patenterteilung: **05.11.2003**
(21) Anmeldenummer: 00909109.1
(22) Anmeldetag: 27.01.2000
(51) Int. Cl.: A61F 2/00, A61L 31/02, A61L 27/04

(54) **OSTEOPHILE IMPLANTATE**
OSTEOPHILIC IMPLANTS
IMPLANTS OSTEOPHILES

(30) Priorität: 29.01.1999 EP 99810078
(43) Veröffentlichungstag der Anmeldung: 07.11.2001
(73) Patentinhaber: Institut Straumann AG, 4437 Waldenburg (CH)
(72) Erfinder: STEINEMANN, Samuel, G., CH-1025 St. Sulpice (CH); SIMPSON, James, CH-4458 Eptingen (CH)
(74) Vertreter: Hepp, Dieter
(86) Internationale Anmeldenummer: EP0000619
(87) Internationale Veröffentlichungsnummer: WO00044305

(56) Entgegenhaltungen:
- EP-A- 0 388 576
- EP-A- 0 606 566
- WO-A-97/26026
- US-A- 4 712 681
- US-A- 4 763 788
- US-A- 5 853 561
- US-A- 5 855 612
- TAYLOR G C ET AL: "Influence of titanium oxide and titanium peroxy gel on the breakdown of hyaluronan by reactive oxygen species" BIOMATERIALS, Bd. 17, Nr. 13, 1. Januar 1996 (1996-01-01), Seite 1313-1319 XP004032646

## Beschreibung

Die vorliegende Erfindung betrifft osteophile Implantate, welche zum Einsetzen in Knochen dienen und welche erheblich verbesserte Osteointegrationseigenschaften aufweisen, sowie Verfahren zu deren Herstellung.

Implantate, welche zum Einsetzen in Knochen dienen, wie beispielsweise Hüft- oder Kniegelenkprothesen oder in den Kiefer einzuschraubenden Stifte für den Aufbau künstlicher Zähne, sind an sich bekannt. Solche Implantate bestehen vorzugsweise aus Titan oder Titanbasislegierungen, wie z.B. Titan/Zirkonlegierungen, wobei diese zusätzlich Niob, Tantal oder andere gewebeverträglichen metallische Zusätze enthalten können. Eine zentrale Eigenschaft solcher Implantate ist deren Osteointegrationszeit, das heisst die Zeit, die vergeht, bis sich die Knochensubstanz in genügender Stärke und dauerhaft mit der Implantatoberfläche verbunden, das heisst integriert, hat.

Wie fest das Implantat im Knochen verankert ist, kann mit mechanischen Messungen festgestellt werden, nämlich durch Messung der Kraft, sei es als Zug, Druck, Scherung oder Drehmoment, welche nötig sind, um das im Knochen verankerte Implantat aus seiner Verankerung herauszuziehen oder heraus zu drehen, d.h. einen Adhäsionsbruch zwischen der Oberfläche des Implantats und der mit dieser verbundenen Knochensubstanz herbeizuführen. Solche Messmethoden sind an sich bekannt und beispielsweise in Brunski, Clinical Materials, Vol. 10, 1992, pp. 153-201, beschrieben. Messungen haben gezeigt, dass sich Titan-Implantate mit glatter Oberflächenstruktur nur ungenügend im Knochen verankern, während Implantate mit aufgerauhter Oberfläche einen bezüglich der Zugfestigkeit merklich verbesserten Knochen-Implantat-Verbund ergeben.

In EP 0 388 576 wird deshalb vorgeschlagen, auf der Implantatoberfläche in einem ersten Schritt mittels Sandstrahlen eine Makrorauhigkeit aufzubringen und diese anschliessend mittels Behandlung in einem Säurebad mit einer Mikrorauhigkeit zu überlagern. So kann die Implantatoberfläche mittels Sandstrahlen aufgerauht und anschliessend mit einem Aetzmittel, z.B. Fluorwasserstoffsäure oder Chlorwasserstoffsäure/Schwefelsäuregemisch behandelt werden. Die so mit einer definierten Rauhigkeit versehene Oberfläche wird dann mit Wasser und Lösungsmitteln gewaschen und einer Sterilisationsbehandlung unterzogen.

In EP 0 606 566 wird vorgeschlagen, die Implantatoberfläche im Vakuum zuerst mit einem inerten Plasmagas, um die Oberfläche zu reinigen, und anschliessend mit einem oxydierend wirkenden Plasmagas oder mittels thermischer Oxydation sowie mit allfällig weiteren Verfahrensschritten zu behandeln, worauf das Implantat in Glasampullen verschlossen und sterilisiert wird. Vorzugsweise werden alle diese Schritte unter Vakuum ausgeführt. Im genannten Verfahren wird das Wachstum eines Oberflächenoxids erzeugt. Diese Oberfläche ist zwar rein, jedoch wird sie in Luftatmosphäre sofort vergiftet bzw. weitgehend chemisch inaktiviert.

Der chemische Zustand der Oberfläche von Titan und Titanbasislegierungen ist komplex. Es wird davon ausgegangen, dass die Oberfläche von Titanmetall in Luft und Wasser spontan oxydiert und dass dann an der Oberfläche, das heisst in der äussersten Atomschicht, eine Reaktion mit Wasser abläuft, wobei Hydroxylgruppen gebildet werden. Diese, Hydroxylgruppen enthaltende, Oberfläche wird in der Literatur als "hydroxylierte" Oberfläche bezeichnet. Siehe H.P. Boehm, Acidic and Basic Properties of Hydroxylated Metal Oxide Surfaces, Discussions Faraday Society, Vol. 52, 1971, pp. 264-275. Eine Metalloberfläche, deren Hydroxylgruppen nicht frei verfügbar sind, weil diese z.B. chemisch verändert wurden, ist im Sinne der vorliegenden Erfindung keine "hydroxylierte" Oberfläche.

Im Sinne der vorliegenden Erfindung wird die metallische Implantatoberfläche als "hydrophil" bezeichnet, wenn sie für die Körperflüssigkeit frei zugänglich ist und nicht von Fremdsubstanzen, beispielsweise von hydrophobierend wirkenden Substanzen, überlagert ist. So sind üblicherweise verschiedene leichtflüchtige Kohlenwassserstoffe in nicht-gereinigter Luft vorhanden. Diese werden von hydroxylierten und hydrophilen Titanmetalloberflächen in kurzer Zeit in dünner Schicht adsorbiert, so dass solche Oberflächen nicht mehr hydrophil sind. Ebenso kann eine solche hydroxylierte und hydrophile Oberfläche hydrophob werden, wenn sich die an der Oberfläche vorhandenen Hydroxylgruppen mit z.B. in der Luft vorhandenem Kohlenstoffdioxid oder im Reinigungsverfahren eingebrachten organischen Lösungsmitteln, wie z.B. Methanol oder Aceton, assoziieren oder chemisch reagieren.

Es wurde nun gefunden, dass eine hydroxylierte und hydrophile Oberfläche von Titan oder einer Titanlegierung biologisch wirksame Eigenschaften aufweist, bzw. biologisch aktiv ist. Man kann diese Oberfläche auch als pseudo-biologisch aktiv bezeichnen. Im weiteren wird der Begriff biologisch aktiv verwendet. Es hat sich überraschenderweise gezeigt, dass eine solche biologisch aktive Oberfläche, z.B. in Form eines Implantats, erheblich schneller mit der Knochensubstanz zu einem starken Verbund zusammen wächst als eine gleiche jedoch nicht-hydroxylierte und/oder nicht-hydrophile Oberfläche. Weiter hat sich gezeigt, dass die biologische Aktivität einer solchen metallischen Oberfläche in Gegenwart von Kohlenstoffdioxid oder flüchtigen Kohlenwasserstoffen, das heisst im Kontakt mit normaler Luft, beispielsweise im Trocknungsprozess, oder beim Reinigen mit Alkohol, sehr schnell verloren geht. Weiter wurde gefunden, dass sich die biologische Aktivität der erwähnten hydroxylierten und hydrophilen Oberfläche weitgehend unverändert erhalten lässt, wenn man diese Oberfläche gemäss der vorliegenden Erfindung behandelt, wie dies im weiteren beschrieben ist. Derart wird die biologische Aktivität der Implantatoberfläche bis zur Implantation des Implantats erhalten.

Die vorliegende Erfindung ist in den Patentansprüchen definiert. Insbesondere betrifft die vorliegende Erfindung ein osteophiles Implantat mit verbesserten Osteointegrationseigenschaften bzw. mit verbesserter Osteointegration, wobei dieses Implantat aus Titan oder einer Titanlegierung besteht und geeignet ist für die Implantation in Knochen, dadurch gekennzeichnet, dass das Implantat eine aufgerauhte, hydroxylierte und hydrophile, und somit biologisch aktive, Oberfläche aufweist.

Vorzugsweise ist diese Oberfläche in einer gas- und flüssigkeitsdichten Umhüllung verschlossen, wobei sich im Innern der Umhüllung keine Verbindungen befinden, welche die biologische Aktivität der Implantatoberfläche beeinträchtigen können.

Vorzugsweise ist das Innere der Umhüllung mindestens teilweise mit reinem Wasser, welches gegebenenfalls weitere Zusatzstoffe enthalten kann, befüllt, wobei mindestens eine solche Menge Wasser anwesend ist, dass die Feuchthaltung bzw. Benetzung der aufgerauhten Implantatoberfläche gewährleistet ist. Das Restvolumen innerhalb der Umhüllung kann mit gegenüber der Implantatoberfläche inerten Gasen, wie z.B. Sauerstoff, Stickstoff, Edelgase oder einem Gemisch solcher Gase befüllt sein.

Die vorliegende Erfindung betrifft auch Verfahren zur Herstellung der erfindungsgemässen Implantate.

Vorzugsweise bestehen die erfindungsgemässen Implantate aus einer Titanlegierung, vorzugsweise aus einer Titan/Zirkonlegierung, wobei diese zusätzlich Niob, Tantal oder andere gewebeverträgliche metallische Zusätze enthalten können. Diese Implantate dienen vorzugsweise als Hüft- oder Kniegelenkprothesen oder als in den Kiefer einzuschraubenden Stifte für den Aufbau künstlicher Zähne. Solche Implantate, deren Beschaffenheit und die für deren Herstellung verwendeten metallischen Materialien sind an sich bekannt und beispielsweise in J. Black, G. Hastings, Handbook of Biomaterials Properties, Seiten 135-200, Verlag Chapman & Hall, London, 1998, beschrieben.

Die strukturelle und funktionelle Verankerung, z.B. eines Zahnimplantats, im Knochen wird in der Regel durch Anbringen einer Makrorauhigkeit, wie eines Schraubengewindes oder von Vertiefungen in der Oberfläche und/oder einer, gegebenenfalls zusätzlichen, Mikrorauhigkeit, erreicht, wobei die Mikrorauhigkeit entweder in einem additiven Prozess mittels Plasmatechnik, oder in einem subtraktiven Prozess durch chemische Ätzung auf der Oberfläche angebracht wird. Wie fest das Implantat im Knochen verankert ist, kann mit mechanischen Messungen festgestellt werden, wie dies bereits eingangs in diesem Text beschrieben wurde. Zahlreiche Untersuchungen haben gezeigt, dass die genügende Verankerung eines Implantat im Knochen in hohem Mass von der Oberflächenbeschaffenheit des Implantats, insbesondere von der Rauhigkeit, abhängt. Bemerkenswert ist, dass die Verwendung einer biologisch aktiven Implantatoberfläche gemäss der vorliegenden Erfindung weitgehend unabhängig ist von der physikalischen Oberflächenbeschaffenheit des Implantats. Gemäss der vorliegenden Erfindung wird die biologische Wirkung der hydroxylierten und hydrophilen Oberfläche zur im wesentlichen physikalischen Wirkung der Oberflächenrauhigkeit synergetisch hinzugefügt, woraus eine erhebliche Verbesserung der Osteointegration resultiert. Die beiden Effekte sind gemäss der vorliegenden Erfindung insofern miteinander verknüpft, als durch eine physikalisch grössere Oberfläche auch mehr biologisch wirksame Oberfläche zur Verfügung steht. Die vorliegende Erfindung zeigt, dass nicht nur die Oberflächenrauhigkeit an sich, sondern auch die chemische Beschaffenheit der Oberfläche die Osteointegration massgeblich beeinflussen.

Analysen von klinisch benutzten Implantatoberflächen (vor deren Implantation) mittels XPS (X-ray excited Photoelectron Spectroscopy) und Auger Elektronen Spektroskopie (AES) wiesen auf eine Verunreinigung der Oberfläche mit Kohlenstoff hin. Benetzungsversuche ergaben einen Benetzungswinkel mit Wasser von 70° und höher (=70°), das heisst eine Wasser abweisende Oberfläche mit hydrophobem Charakter. Weitere Untersuchungen ergaben, dass solche Oberflächen biologisch inaktiv sind. Überraschenderweise wurde festgestellt, dass die Oberfläche, wie sie z.B. direkt nach der Säureätzung anfällt und hydroxyliert und hydrophil ist, einen Benetzungswinkel mit Wasser von weniger als 50° (<50°) bei Vordringen des Wassertropfens im Kontakt mit der Oberfläche, respektive von weniger als 20° (<20°) im Falle des sich zurückbildenden Tropfens, hat und eine bemerkenswerte biologische Aktivität aufweist, wobei diese im wesentlichen erhalten bleibt, wenn auf weitere Reinigungs- und Sterilisationsverfahren verzichtet und ein Kontakt der Implantatoberfläche mit der Luft vermieden wird.

Die vorliegenden Erfindung beruht auf der biologischen Wirkung bezüglich der Osteointegration von Implantaten, welche aus Titan oder Titanlegierungen hergestellt sind, indem diese Implantate eine hydroxylierte und hydrophile Oberfläche und gleichzeitig -infolge der vorhandenen Rauhigkeit - eine vergrösserte wirksame Oberfläche aufweisen. Diese biologisch wirksame Oberfläche kann z.B. durch mechanische Bearbeitung und Strukturierung, Kugelstrahlen, Sandstrahlen und anschliessender chemischer Behandlung, z.B. Ätzung mit Säure; oder mittels der Verwendung elektrochemischer (elektrolytischer) Behandlung oder durch eine Kombination solcher Verfahren hergestellt werden.

Die erfindungsgemässe Oberfläche kann beispielsweise hergestellt werden, indem man die Oberfläche mit der gewünschten Rauhigkeit bzw. Textur versieht und die dabei allenfalls entstehende hydroxylierte und hydrophile Oberfläche in dem erhaltenen Zustand bewahrt oder die aufgerauhte und behandelte Oberfläche in einem separaten Verfahrensschritt in einen hydroxylierten und hydrophilen Zustand bringt. Insbesondere kann man das Implantat herstellen, indem man die Implantatoberfläche kugelstrahlt, sandstrahlt und/oder unter Verwendung von Plasmatechnik aufrauht, und anschliessend die mechanisch aufgerauhte Oberfläche mit einem elektrolytischen oder chemischen Prozess behandelt bis eine hydroxylierte und hydrophile Oberfläche entstanden ist. Vorzugsweise ätzt man das Implantat mit einer anorganischen Säure oder einem Gemisch anorganischer Säuren, vorzugsweise mit Fluorwassserstoffsäure, Chlorwasserstoffsäure, Schwefelsäure, Salpetersäure oder einem Gemisch solcher Säuren oder aber die Oberfläche wird mit Chlorwasserstoffsäure, Wasserstoffperoxid und Wasser im Gewichtsverhältnis von etwa 1:1:5, aktiviert. Anschliessend wird die Oberfläche mit reinem Wasser in inerter Atmosphäre gewaschen.

Vorzugsweise geht man so vor, dass man
- das Implantat kugelstrahlt und anschliessend mit verdünnter Fluorwassserstoffsäure bei Raumtemperatur ätzt; oder
- das Implantat sandstrahlt, z.B. mit Aluminiumoxid Partikeln mit einer durchschnittlichen Korngrösse von 0.1-0.25 mm oder 0.25-0.5 mm und anschliessend mit einem Chlorwasserstoffsäure/-Schwefelsäuregemisch bei erhöhter Temperatur behandelt und mit reinem destilliertem und CO₂-freiem Wasser wäscht; oder
- das Implantat mit grobem Korn sandstrahlt, z.B. mit einem Korngemisch wie vorgängig definiert, und anschliessend mit einem Chlorwasserstoffsäure/Salpetersäuregemisch behandelt und mit reinem destilliertem und CO₂-freiem Wasser wäscht; oder
- das Implantat mit einem Gemisch von Chlorwasserstoff, Wasserstoffperoxid und Wasser im Gewichtsverhältnis von etwa 1:1:5 behandelt und mit reinem destilliertem und CO₂-freiem Wasser wäscht; oder
- das Implantat mittels der Verwendung von Plasmatechnik aufrauht und anschliessend in einem Gemisch von Chlorwasserstoff, Wasserstoffperoxid und Wasser im Gewichtsverhältnis von etwa 1:1:5 hydroxyliert und mit reinem destilliertem und CO₂-freiem Wasser wäscht; oder
- das Implantat in einem elektrolytischen Verfahren behandelt, wobei die Oberfläche gegebenenfalls vorgängig mechanisch aufgerauht wurde, und anschliessend mit reinem destilliertem und CO₂-freiem Wasser wäscht.

In allen Fällen wird das Implantat erfindungsgemäss nicht weiter nachbehandelt, das heisst, es findet keine Behandlung mit Alkohol, Aceton oder einem anderen organischen Lösungsmittel, statt. Insbesondere enthält dieses reine Wasser weder Kohlendioxid noch Dämpfe von Kohlenwasserstoffen und insbesondere kein Aceton und keine Alkohole, wie Methanol oder Ethanol. Es kann aber spezielle Zusatzstoffe enthalten, wie dies im weiteren beschrieben ist. Das zum Waschen verwendete "reine" Wasser ist vorzugsweise mehrfach destilliertes oder via inverse Osmose hergestelltes Wasser, welches vorzugsweise in inerter Atmosphäre, das heisst z.B. unter erniedrigtem Druck, in Stickstoff- oder Edelgasatmosphäre hergestellt wurde.

Im weiteren hat das reine Wasser eine elektrischen Widerstand von mindestens 2 Mohmcm (elektrischer Widerstand >2 Mohmcm) und einen Gesamtgehalt an organischem Kohlenstoff (total organic carbon, TOC) von höchstens 10 ppb (≤10 ppb).

Anschliessend an den Waschprozess wird das erhaltene Implantat in reinem Wasser belassen und in einem geschlossenem Gefäss bzw. Umhüllung aufbewahrt. Neben Wasser kann das Innere der Umhüllung inerte Gase, beispielsweise Stickstoff, Sauerstoff oder Edelgas, wie z.B. Argon, enthalten. Vorzugsweise wird das erhaltene Implantat in reinem Wasser, welches gegebenenfalls mit ausgewählten Zusatzstoffen versehen ist, und in einer Umhüllung, welche praktisch für Gase und Flüssigkeiten undurchlässig ist, insbesondere für Kohlenstoffoxide, aufbewahrt, wobei sich im Innern der Umhüllung keine Verbindungen befinden, welche die biologische Aktivität der Implantatoberfläche beeinträchtigen können.

Verbindungen, welche die biologische Aktivität der Implantatoberfläche beeinträchtigen können, sind beispielsweise, wie bereits erwähnt, Methanol, Ethanol, Aceton und verwandte Ketone sowie zahlreiche anderen organische Verbindungen, oder Kohlendioxid.

In diesem Sinn betrifft die vorliegende Erfindung auch ein Verfahren zur Herstellung eines erfindungsgemässen Implantats, indem man die Implantatoberfläche kugelstrahlt, sandstrahlt und/oder unter Verwendung von Plasmatechnik aufrauht, dadurch gekennzeichnet, dass man anschliessend
(i) die mechanisch oder plasmatechnisch aufgerauhte Oberfläche mit einem elektrolytischen oder chemischen Ätzverfahren behandelt bis eine hydroxylierte und hydrophile Oberfläche entstanden ist, vorzugsweise mit einer anorganischen Säure oder einem Gemisch anorganischer Säuren, vorzugsweise mit Fluorwasserstoffsäure, Chlorwasserstoffsäure, Schwefelsäure, Salpetersäure, oder einem Gemisch solcher Säuren, oder Chlorwasserstoff, Wasserstoffperoxid und Wasser im Gewichtsverhältnis von etwa 1:1:5;
(ii) die Oberfläche mit reinem Wasser, welches gegebenenfalls weitere Zusatzstoffe enthält, in inerter Atmosphäre wäscht, und
(iii) die Oberfläche, unter Verzicht auf eine weitere Behandlung, in inerter Atmosphäre und dauernd in Gegenwart von reinem Wasser, welches gegebenenfalls weitere Zusatzstoffe enthält, aufbewahrt, vorzugsweise in einer Umhüllung, welche praktisch für Gase und Flüssigkeiten undurchlässig ist.

Die negative Wirkung von Methylalkohol kann z.B. damit erklärt werden, dass Methylalkohol mit der sich an der Oberfläche befindenden Hydroxylgruppe eine Kondensationsreaktion gemäss der Formel: ≡TiOH + CH₃OR → ≡TiOCH₃ + H₂O eingeht, wobei ≡Ti- ein Metallion an der Metalloberfläche bedeutet. Kohlendioxid z.B. reagiert mit der Hydroxidgruppe unter Bildung eines Bikarbonatkomplexes, der die Hydroxylgruppe inaktiviert. Analogerweise kann die Reaktion einer organischen Carbonsäure mit der Hydroxylgruppe erklärt werden. Im weiteren kann man der Oberfläche in Abhängigkeit des Säurewertes des die Oberfläche umgebenden Elektrolyten einen amphoteren Charakter zuschreiben, wobei eine Wechselwirkung zwischen der Säure im Elektrolyt und dem basisch reagierenden Hydroxyl bzw. dem Anion im Elektrolyt und dem sauer reagierenden Hydroxyl des Oxids besteht. Zur Erklärung der Oberflächenreaktionen können die Bildung von kovalenten Bindungen, elektrostatische Effekte und/oder die Bildung von Wasserstoffbrücken herangezogen werden. In diesem Sinn wird davon ausgegangen, dass die Hydroxylgruppen an der Implantatoberfläche die eigentlichen Träger der biologischen Aktivität sind und direkt auf die Knochenmineralien und die organische Knochensubstanz einwirken, mit welchen sie nach der Implantierung des Implantats in Kontakt treten und mit diesen reagieren.

Die vorliegende Erfindung ist aber nicht an diese Erklärungen gebunden. Entscheidend ist die Tatsache, dass gewisse Verbindungen die biologische Aktivität einer hydroxylierten und hydrophilen erfindungsgemässen Oberfläche negativ beeinflussen und diese Aktivität bezüglich der biologischen Körpersubstanz vermindern oder gänzlich desaktivieren. Der Fachmann kann experimentell feststellen, ob und wie weit eine Verbindung die biologische Aktivität negativ beeinflusst, beispielsweise wie dies u.a. hierin im weiteren beschrieben ist.

Vorzugsweise ist das erfindungsgemässe Implantat, mindestens jedoch dessen hydroxylierte und hydrophile Oberfläche in einer gas- und flüssigkeitsdichten Umhüllung verschlossen, wobei sich im Innern der Umhüllung keine Verbindungen befinden, welche die biologische Aktivität der Implantatoberfläche beeinträchtigen können. Diese gas- und flüssigkeitsdichte Umhüllung ist vorzugsweise eine verschweisste Ampulle aus Glas, Metall, einem synthetischen Polymeren oder einem anderen gas- und flüssigkeitsdichten Material oder einer Kombination dieser Materialien darstellt. Das Metall liegt vorzugsweise als dünne Metallfolie vor, wobei polymere Materialien und metallische Folien, aber auch Glas, in an sich bekannter Weise miteinander zu einer geeigneten Verpackung kombiniert werden können.

Vorzugsweise ist das Innere der Umhüllung mindestens teilweise mit gereinigtem Wasser, welches gegebenenfalls weitere Zusatzstoffe enthalten kann, befüllt, wobei mindestens eine solche Menge Wasser anwesend ist, dass die Feuchthaltung der Implantatoberfläche gewährleistet ist. Überraschenderweise hat sich gezeigt, dass die Feuchthaltung der erfindungsgemässen Implantatoberfläche mit reinem Wasser deren chemischen und biologisch aktiven Zustand stabilisiert und über eine längere Zeit, in der Regel bis zur Implantation, aufrecht erhält. Im weiteren hat sich gezeigt, dass die Osteointegrationseigenschaften der Oberfläche durch geeignete Zusätze noch verbessert werden können. In diesem Sinn werden beispielsweise die Zelladhäsion an die Implantatoberfläche verbessert und die Verankerungszeit des Implantats im Knochen verkürzt. Man könnte auch sagen, die Implantatoberfläche hat osteophile (oder osseophile) Eigenschaften, welche durch die geeigneten Zusätze verbessert werden.

Geeignete Zusätze, welche dem reinem Wasser erfindungsgemäss zugesetzt werden können, sind beispielsweise einwertige Alkalikationen, wie Na⁺ oder K⁺, oder ein Gemisch von Na⁺ und K⁺, mit entsprechenden Anionen in Form anorganischer Salze, wie z.B. Natriumchlorid, Kaliumchlorid, Natrium- oder Kaliumchlorat, Natrium- oder Kaliumnitrat, Natrium- oder Kaliumphosphat oder ein Gemisch solcher Salze. Ebenso können auch zweiwertige Kationen mit Form von wassserlöslichen anorganischen Salzen zugesetzt werden. Geeignete Kationen sind insbesondere Mg⁺², Ca⁺², Sr⁺² und/oder Mn⁺² in Form der Chloride oder deren Gemische. Geeignete Anionen sind auch Phosphat- und Phosphonatanionen, wobei darunter jeweils auch Monoorthophosphat-Anionen und Diorthophosphat-Anionen bzw. Monoorthophosphonat-Anionen und Diorthophosphonat-Anionen zu verstehen sind, in Kombination mit den genannten Kationen.

Bevorzugt sind Kationen und Anionen, welche bereits in der Körperflüssigkeit vorkommen, insbesondere in der jeweiligen physiologischen Konzentration und bei einem physiologischen Säurewert (pH-Wert) im Bereich von vorzugsweise 4 bis 9 und vorzugsweise bei einem Säurewert im Bereich von 6 bis 8. Bevorzugte Kationen sind Na⁺, K⁺, Mg⁺² und Ca⁺². Das bevorzugte Anion ist Cl⁻. Bevorzugt liegt die Gesamtmenge der genannten Kationen bzw. Anionen jeweils im Bereich von etwa 50 mEq/l bis 250 mEq/l, vorzugsweise etwa 100 mEq/l bis 200 mEq/l und vorzugsweise bei etwa 150 mEq/l. Dabei bedeutet Eq/l (Formel-)Equivalentgewicht bzw. Eq/l entspricht dem Atomgewicht der Formeleinheit geteilt durch die Wertigkeit. mEq/l bedeutet Milliäquivanlentgewicht pro Liter. Enthält die Umhüllung zweiwertige Kationen, insbesondere Mg⁺², Ca⁺², Sr⁺² und/oder Mn⁺², alleine oder in Kombination mit den erwähnten einwertigen Kationen, so liegt die Gesamtmenge der anwesenden zweiwertigen Kationen vorzugsweise im Bereich von 1 mEq/l bis 20 mEq/l.

Die erfindungsgemässe Belegung der Implantatoberfläche mit Wasser hat den weiteren Vorteil, dass die hydroxylierte und hydrophile Implantatoberfläche bei der Entnahme aus der Umhüllung, durch die wässrige Belegung der Oberfläche mit Wasser, welches gegebenenfalls ausgewählte Kationen und Anionen enthält, temporär vor der Einwirkung von schädlichen in der Luft enthaltenen Substanzen, geschützt ist.

Methoden zur Messung von Metalloberflächen, im vorliegende Fall der effektiven Oberfläche der aufgerauhten und mit einer Oberflächentextur versehen Implantatoberfläche sind an sich bekannt. So sind beispielsweise elektrochemische Messmethoden bekannt, welche ausführlich in P.W. Atkins, Physical Chemistry, Oxford University Press, 1994, beschrieben sind. Die Oberfläche kann bestimmt werden durch Messung (a) der elektrophoretischen Beweglichkeit, (b) der Oberflächenladung mittels Säure-Base Titration, mittels (c) Impedanzspektrometrie, oder (d) mittels Voltametrie. Auch aus Rauhigkeitsmessungen kann die effektive Oberfläche als Quadrat des hybriden Parameters Lᵣ, d.h. dem Quadrat des Profillängenverhältnis erhalten werden. In der Norm DIN 4762 ist der Parameters Lᵣ definiert als das Verhältnis der Länge des gestreckten zweidimensionalen Profils und der vermessenen Distanz. Letztere Messung hat aber zur Voraussetzung, dass die vertikale und laterale Auflösung kleiner ist als 1µm und sogar nahe bei 0.1µm liegt.

Die Referenzfläche für alle diese Messmethoden ist die flache polierte Metalloberfläche. Die gemessenen Werte der aufgerauhten Oberfläche im Vergleich zu den an der flachen und polierten Oberfläche, geben an, um wieviel grösser die aufgerauhte Oberfläche ist, verglichen mit der flachen und polierten Oberfläche. In vitro Untersuchungen mit Knochenzellen und in vivo histomorphometrische Untersuchungen an erfindungsgemässen Implantaten weisen darauf hin, dass die osteophilen Eigenschaften der erfindungsgemässen Implantate besonders hoch sind, wenn die aufgerauhte Oberfläche vorzugsweise mindestens 1.5 mal und vorzugsweise mindestens zweimal so gross ist, wie die vergleichbare flache und polierte Oberfläche. Bevorzugt ist die aufgerauhte Implantatoberfläche mindestens 2 bis 12 mal so gross, und vorzugsweise etwa 2.5 bis 6 mal so gross, wie die vergleichbare flache und polierte Oberfläche.

Die Benetzungseigenschaften bzw. der hydrophile Charakter der erfindungsgemässen Implantatoberfläche kann durch Messung des Kontaktwinkels bzw. Benetzungswinkels zwischen der Flüssigkeit (Wasser) und der trockenen metallischen Substratoberfläche mittels optischer Methoden in an sich bekannter Weise bestimmt werden. Für die Bestimmung des Kontaktwinkels einer erfindungsgemässen Implantatoberfläche, wird diese mit reinem Wasser gewaschen und in reinem Stickstoff oder reinem Argon getrocknet. Ein Tropfen reinen Wassers wird auf die horizontal ausgerichtete Oberfläche gegeben. Durch Hinzufügen weiteren Wassers, z.B. mit einer Hohlnadel, wird die Tropfenoberfläche vergrössert, was den "oberen" Kontaktwinkel ergibt, während die Entnahme von Wasser den Tropfendurchmesser im Kontakt mit der Oberfläche verkleinert, was den "unteren" Kontaktwinkel ergibt. Ein hydrophiler Charakter der Oberfläche ist gegeben, wenn der "obere" Kontaktwinkel kleiner als 50° (<50°) ist und der "untere" Kontaktwinkel weniger als 20° (<20°) beträgt.

Industriell hergestellte Oberflächen von Titan und Titanlegierungen für die Bearbeitung in Laboratorien und Kliniken weisen in der Regel Verunreinigungen auf, welche im wesentlichen aus Kohlenstoffverbindungen und Spuren von Stickstoff, Kalzium, Schwefel, Phosphor und Silizium bestehen. Diese Verunreinigungen konzentrieren sich in der äussersten Metalloxidschicht. Vorzugsweise enthält die hydroxylierte und hydrophile Implantatoberfläche höchstens 20 Atom-% Kohlenstoff, gemessen mit spektroskopischen Methoden, wie XPS oder AES oder andere an sich bekannten spektroskopischen Methoden.

Die folgenden Beispiele erläutern die Erfindung.

### Beispiel 1

Eine gängige Form eines Zahnimplantats in Form einer Schraube von 4 mm Durchmesser und 10 mm Länge wurde hergestellt. Die Rohform wurde zerspanend durch Drehen und Fräsen des zylindrischen Rohlings in an sich bekannter Weise erhalten. Die in den Knochen einzusetzende Oberfläche wurde nun gemäss EP 0 388 576 mit einer Makrorauhigkeit versehen, indem diese mit einem Korn der mittleren Korngrösse 0.25-0.5 mm sandgestrahlt wurde. Anschliessend wurde die aufgerauhte Oberfläche (Makrorauhigkeit) mit einem wässerigen Chlorwasserstoffsäure/Schwefelsäuregemisch mit einem Verhältnis von HCl:H₂SO₄:H₂O von 2:1:1 bei einer Temperatur von über 80°C während etwa fünf Minuten behandelt, so dass ein Verhältnis der aufgerauhten Implantatoberfläche zur vergleichbaren polierten Oberfläche von 3.6, gemessen mittels Voltametrie im wässerigen Elektrolyten mit 0.15M NaCl, (entsprechend einem Verhältnis von 3.9, gemessen mit Impedanzspektrometrie im 0.1 molaren Na₂SO₄-Elektrolyten), erhalten wurde. Das so geformte Implantat wurde mit reinem Wasser gewaschen. Anschliessend wurde das Implantat
a) direkt in einer Glasampulle, welche mit reinem Wasser gefüllt war, verschweisst, nach 4 Wochen geöffnet und implantiert;
b) direkt in einer Glasampulle verschweisst, welche gefüllt war mit reinem Wasser, enthaltend 150 mEq/l an Na⁺-Ionen, und 10 mEq/l an Mg⁺²-Ionen sowie die entsprechende Menge Cl⁻Anionen, und nach 4 Wochen geöffnet und implantiert;
c) mit CO₂-enthaltender Luft getrocknet und implantiert (Vergleichsversuch).

Die gemäss den Versuchen a), b) und c) erhaltenen Implantate wurden im Oberkiefer eines Minischweins implantiert. Die Verankerung im Knochen wurde als Lösedrehmoment der im Oberkiefer des Minischweins implantierten Schraube gemessen. Die erhaltenen Ergebnisse sind in Tabelle 1 angegeben.

**Tabelle 1**

| | Verankerung* nach 2 Wochen (Ncm) | Verankerung* nach 3 Wochen (Ncm) | Verankerung* nach 4 Wochen (Ncm) |
|---|---|---|---|
| Versuch a) | 35 | 72 | 100 |
| Versuch b) | 55 | 80 | 110 |
| Vergleichs-versuch c) | 20 | 58 | 80 |

| | | | |
|---|---|---|---|
| * die Verankerung ist als Lösedrehmoment in Ncm (Durchschnittswerte) angegeben. | | | |

Die Ergebnisse gemäss den Versuchen a) und b) (erfindungsgemässe Implantate) zeigen, dass die entsprechenden Lösedrehmomente für die angegebenen Einheilzeiten deutlich über denjenigen von Versuch c) liegen.

### Beispiel 2

Beispiel 1, Versuche b) und c) wurden wiederholt, jedoch mit der Massgabe, dass ein Implantat mit einem Verhältnis der aufgerauhten Implantatoberfläche zur vergleichbaren polierten Oberfläche von 1.9 (gemessen mit Impedanzspektrometrie im 0.1 molaren Na₂SO₄-Elektrolyten) hergestellt wurde. Hierzu wurde die Implantatoberfläche nur mechanisch, durch Drehen, geschnitten und anschliessend wie in Beispiel 1 angegeben geätzt. Das so erhaltene Implantat wurde mit reinem Wasser gewaschen. Anschliessend wurde das Implantat
d) direkt in einer Glasampulle verschweisst, welche gefüllt war mit reinem Wasser, enthaltend 150 mEq/l an Na⁺-Ionen, und 10 mEq/l an Mg⁺²-Ionen sowie die entsprechende Menge Cl⁻Anionen, und nach 4 Wochen geöffnet und implantiert;
e) mit CO₂-enthaltender Luft getrocknet und implantiert (Vergleichsversuch).

Die gemäss den Versuchen d) und e) erhaltenen Implantate wurden im Oberkiefer eines Minischweins implantiert. Die Verankerung im Knochen wurde als Lösedrehmoment der im Oberkiefer des Minischweins implantierten Schraube gemessen. Die erhaltenen Ergebnisse sind in Tabelle 2 angegeben.

**Tabelle 2**

| | Verankerung* nach 2 Wochen (Ncm) | Verankerung* nach 3 Wochen (Ncm) | Verankerung* nach 4 Wochen (Ncm) |
|---|---|---|---|
| Versuch d) | 10 | 40 | 65 |
| Vergleichs-versuch e) | 5 | 25 | 60 |

| | | | |
|---|---|---|---|
| * die Verankerung ist als Lösedrehmoment in Ncm (Durchschnittswerte) angegeben. | | | |

Die Ergebnisse gemäss Versuch d)(erfindungsgemässes Implantat) zeigen, dass die entsprechenden Lösedrehmomente für die angegebenen Einheilzeiten deutlich über denjenigen von Versuch c) liegen. Geht man davon aus, dass in der Zahnchirurgie für den Aufbau der Suprastruktur ein Lösedrehmoment von mindestens 35 Ncm als unbedingt nötig erachtet wird, so wird dieser Wert vom erfindungsgemässen Implantat spätestens nach 3 Wochen erreicht.

## Patentansprüche

1. Osteophiles Implantat , welches aus Titan oder einer Titanlegierung besteht und geeignet ist für die Implantation in Knochen, **dadurch gekennzeichnet, dass** (i) das Implantat eine aufgerauhte, hydroxylierte und hydrophile Oberfläche aufweist, und (ii) mindestens dessen hydroxylierte und hydrophile Oberfläche in einer gas- und flüssigkeitsdichten Umhüllung verschlossen ist, und diese Umhüllung im Innenraum reines Wasser, welches gegebenenfalls mit ausgewählten zusatzstoffen versehen ist, eine gegenüber der Implantatoberfläche und/oder inerte Atmosphäre aufweist, vorzugsweise bestehend aus Stickstoff, Sauerstoff und/oder Edelgas.

2. Implantat nach Anspruch 1, **dadurch gekennzeichnet, dass** dieses-aus einer Titan/Zirkonlegierung besteht, welche gegebenenfalls zusätzlich Niob, Tantal oder andere gewebeverträglichen metallische Zusätze enthält.

3. Implantat nach Anspruch 1 oder 2, **dadurch gekennzeichnet, dass** die hydroxylierte und hydrophile Oberfläche mit einem elektrolytischen oder chemischen Ätzverfahren, vorzugsweise durch Behandlung mit einer anorganischen Säure oder einem Gemisch anorganischer Säuren, vorzugsweise mit Fluorwassserstoffsäure, Chlorwasserstoffsäure, Schwefelsäure, Salpetersäure, oder einem Gemisch solcher Säuren, oder Chlorwasserstoffsäure, Wasserstoffperoxid und Wasser im Gewichtsverhältnis von etwa 1:1:5, erhalten wurde.

4. Implantat nach einem des Ansprüche 1-3, **dadurch gekennzeichnet, dass** das Innere der Umhüllung mindestens teilweise mit reinem Wasser, welches gegebenenfalls weitere Zusatzstoffe enthält, befüllt ist, und mindestens eine solche Menge Wasser anwesend ist, dass die Benetzung der aufgerauhten Implantatoberfläche gewährleistet ist und das Restvolumen innerhalb der Umhüllung gegebenenfalls mit gegenüber der Implantatoberfläche inerten Gasen, vorzugsweise mit Sauerstoff, Stickstoff, Edelgase oder einem Gemisch solcher Gase befüllt ist.

5. Implantat nach einem der Ansprüche 1-4, **dadurch gekennzeichnet, dass** das Wasser im Innern der Umhüllung einen elektrischen Widerstand von mindestens 2 Mohmcm und einen Gesamtgehalt an organischem Kohlenstoff von höchstens 10 ppb aufweist.

6. Implantat nach einem der Ansprüche 1-5, **dadurch gekennzeichnet, dass** das Wasser im Innern der Umhüllung einwertige Alkalikationen und/oder zweiwertige Kationen mit Form von wasserlöslichen anorganischen Salzen, enthält.

7. Implantat nach einem der Ansprüche 1-6, **dadurch gekennzeichnet, dass** das Wasser in Innern der Umhüllung als einwertige Alkalikationen Na⁺ oder K⁺, oder ein Gemisch von Na⁺ und K⁺, mit entsprechenden Anionen in Form anorganischer Salze, vorzugsweise als Natriumchlorid, Kaliumchlorid, Natrium- oder Kaliumchlorat, Natrium- oder Kaliumnitrat, Natrium- oder Kaliumphosphat, Natriumoder Kaliumphosphonat oder ein Gemisch solcher Salze enthält.

8. Implantat nach einem der Ansprüche 1-7, **dadurch gekennzeichnet, dass** das Wasser im Innern der Umhüllung als zweiwertige Kationen Mg⁺², Ca⁺², Sr⁺² oder Mn⁺² oder deren Gemische, vorzugsweise Mg⁺² und/oder Ca⁺², in Form der Chloride enthält.

9. Implantat nach einem der Ansprüche 1-8, **dadurch gekennzeichnet, dass** das Wasser im Innern der Umhüllung Kationen und Anionen, welche bereits in der Körperflüssigkeit vorkommen, enthält, vorzugsweise in der jeweiligen physiologischen Konzentration.

10. Implantat nach einem der Ansprüche 1-9, **dadurch gekennzeichnet, dass** das Wasser im Innern der Umhüllung einen Säurewert im Bereich von 4 bis 9, vorzugsweise von 6 bis 8, aufweist.

11. Implantat nach einem der Ansprüche 1-10, **dadurch gekennzeichnet, dass** die Gesamtmenge der Kationen bzw. Anionen im Innern der Umhüllung jeweils im Bereich von etwa 50 mEq/l bis 250 mEq/l, vorzugsweise jeweils im Bereich von etwa 100 mEq/l bis 200 mEq/l und vorzugsweise jeweils bei etwa 150 mEq/l liegt und die Gesamtmenge der anwesenden zweiwertigen Kationen vorzugsweise 1 mEq/l bis 20 mEq/l beträgt.

12. Implantat nach einem der Ansprüche 1-11, **dadurch gekennzeichnet, dass** die aufgerauhte Implantatoberfläche mindestens 1.5 mal, vorzugsweise 2 -12 mal, vorzugsweise mindestens 2.5 bis 6 mal, so gross ist, wie die vergleichbare flache und polierte Oberfläche.

13. Implantat nach einem der Ansprüche 1-12, **dadurch gekennzeichnet, dass** der "obere" Kontaktwinkel der hydrophilen Implantatoberfläche kleiner als 50° und der "untere" Kontaktwinkel kleiner als 20° ist.

14. Implantat nach einem der Ansprüche 1-13, **dadurch gekennzeichnet, dass** die hydroxylierte und hydrophile Implantatoberfläche höchstens 20 Atom-% Kohlenstoff enthalt.

15. Implantat nach einem der Ansprüche 1-14, **dadurch gekennzeichnet, dass** die gas- und flüssigkeitsdichten Umhüllung eine verschweisste Ampulle aus Glas, Metall, einen synthetischen Polymeren oder einem anderen gas- und flüssigkeitsdichten Material oder einer Kombination dieser Materialien darstellt, und das Metall vorzugsweise als dünne Metallfolie vorliegt.

16. Verfahren zur Herstellung eines Implantats nach einen der Ansprüche 1-15, wobei man die Implantatoberfläche kugelstrahlt, sandstrahlt und/oder unter Verwendung von Plasmatechnik aufrauht, und anschliessend
(i) die mechanisch oder plasmatechnisch aufgerauhte Oberfläche mit einem elektrolytischen oder chemischen Ätzverfahren behandelt bis eine hydroxylierte und hydrophile Oberfläche entstanden ist, vorzugsweise mit einer anorganischen Säure oder einem Gemisch anorganischer Säuren, vorzugsweise mit Fluorwasserstoffsäure, Chlorwasserstoffsäure, Schwefelsäure, Salpetersäure, oder einem Gemisch solcher Säuren, oder Chlorwasserstoff, Wasserstoffperoxid und Wasser im Gewichtsverhältnis von etwa 1:1:5;
(ii) die Oberfläche mit reinem Wasser, welches gegebenenfalls Zusatzstoffe enthält, in gegenüber der Implantatoberfläche inerter Atmosphäre wäscht, und
(iii) die oberfläche, unter Verzicht auf eine weitere Behandlung, in gegenüber der Implantatoberfläche inerter Atmosphäre und /oder dauernd in Gegenwart von reinem Wasser, welches gegebenenfalls Zusatzstoffe enthält, aufbewahrt, vorzugsweise in einer Umhüllung, welche praktisch für Gase und Flüssigkeiten undurchlässig ist.

## Claims

1. Osteophilic implant, which comprises titanium or a titanium alloy and is suitable for implantation in bones,
**characterised in that**
(i) the implant has a roughened, hydroxylated and hydrophilic surface,
and
(ii) at least its hydroxylated and hydrophilic surface is sealed in a gas-tight and fluid-tight covering, and this covering has in its interior pure water, which if necessary is equipped with selected additives, and/or an atmosphere that is inert in relation to the implant surface, preferably comprising nitrogen, oxygen and/or noble gas.

2. Implant in accordance with claim 1, **characterised in that** it comprises a titanium / zircon alloy, which if necessary additionally contains niobium, tantalum or other metallic additives that are compatible with tissue.

3. Implant in accordance with claim 1 or 2, **characterised in that** the hydroxylated and hydrophilic surface has been achieved with an electrolytic or chemical etching procedure, preferably through treatment with an inorganic acid or with a blend of inorganic acids, preferably with hydrofluoric acid, hydrochloric acid, sulphuric acid, nitric acid or a blend of such acids, or hydrochloric acid, hydrogen peroxide and water in a weight ratio of approximately 1:1:5.

4. Implant in accordance with one of the claims 1-3, **characterised in that** the interior of the covering is filled at least in part with pure water, which if necessary contains further additives, and at least such a quantity of water is present to ensure wetting of the roughened implant surface, and the remaining volume within the covering is filled, if necessary, with gases which are inert in relation to the implant surface, preferably with oxygen, nitrogen, noble gases or a blend of such gases.

5. Implant in accordance with one of the claims 1-4, **characterised in that** the water in the interior of the covering has an electrical resistance of at least 2 Mohms, and a total content of organic carbon of 10 ppb at most.

6. Implant in accordance with one of the claims 1-5, **characterised in that** the water in the interior of the covering contains monovalent alkali cations and/or bivalent cat ions with the form of water-soluble inorganic salts.

7. Implant in accordance with one of the claims 1-6, **characterised in that** the water in the interior of the covering contains, as monovalent alkali cations, Na⁺ or K⁺ or a blend of Na⁺ or K⁺, with corresponding anions in the form of inorganic salts, preferably as sodium chloride, potassium chloride, sodium chlorate or potassium chlorate, sodium nitrate or potassium nitrate, sodium phosphate or potassium phosphate, sodium phosphonate or potassium phosphonate, or a blend of such salts.

8. Implant in accordance with one of the claims 1-7, **characterised in that** the water in the interior of the covering contains, as bivalent cations, Mg⁺², Ca⁺², Sr⁺² or Mn⁺² or their blends, preferably Mg⁺² and/or Ca⁺², in the form of the chlorides.

9. Implant in accordance with one of the claims 1-8, **characterised in that** the water in the interior of the covering contains cat ions and anions which already occur in body fluid, preferably in the respective physiological concentration.

10. Implant in accordance with one of the claims 1-9, **characterised in that** the water in the interior of the covering has an acid value in the range from 4 to 9, preferably from 6 to 8.

11. Implant in accordance with one of the claims 1-10, **characterised in that** the total quantity of the cations and anions in the interior of the covering is respectively in the range of around 50 mEq/l to 250 mEq/l, preferably respectively in the range of around 100 mEq/l to 200 mEq/l, and preferably respectively around 150 mEq/l, and the total quantity of the bivalent cations present is preferably 1 mEq/l to 20 mEq/l.

12. Implant in accordance with one of the claims 1-11, **characterised in that** the roughened implant surface is at least 1.5 times, preferably 2 - 12 times, preferably at least 2.5 to 6 times, as large as the comparable flat and polished surface.

13. Implant in accordance with one of the claims 1-12, **characterised in that** the "upper" contact angle of the hydrophilic implant surface is less than 50° and the "lower" contact angle is less than 20°.

14. Implant in accordance with one of the claims 1-13, **characterised in that** the hydroxylated and hydrophilic implant surface contains at most 20 atom % of carbon.

15. Implant in accordance with one of the claims 1-14, **characterised in that** the gas-tight and fluid-tight covering represents a welded ampoule of glass, metal, a synthetic polymer or another gas-tight and fluid-tight material or a combination of these materials, and the metal is preferably present in the form of a thin metal foil.

16. Method for the production of an implant in accordance with one of the claims 1 - 15, wherein the implant surface is shot-blasted, sand-blasted and/or roughened using plasma technology, and subsequently
(i) the surface that has been roughened mechanically or by means of plasma technology is treated with an electrolytic or chemical etching procedure until a hydroxylated and hydrophilic surface has been produced, preferably with an inorganic acid or a blend of inorganic acids, preferably with hydrofluoric acid, hydrochloric acid, sulphuric acid, nitric acid or a blend of such acids, or hydrochloric acid, hydrogen peroxide and water in a weight ratio of approximately 1:1:5;
(ii) the surface is washed with pure water which, if necessary, contains additives, in an atmosphere that is inert in relation to the implant surface, and
(iii) the surface, without subjection to a further treatment, is stored in an atmosphere that is inert in relation to the implant surface, and/or constantly in the presence of pure water which, if necessary, contains additives, preferably in a covering which is practically impermeable for gases and liquids.

## Revendications

1. Implant ostéophile qui est constitué de titane ou d'un alliage de titane et qui convient pour l'implantation dans des os, **caractérisé en ce que** (i) il présente une surface rendue rugueuse, hydroxylée et hydrophile et (ii) au moins sa surface hydroxylée et hydrophile est enfermée dans une enveloppe imperméable aux gaz et aux liquides et cette enveloppe présente, dans l'espace intérieur, de l'eau pure contenant éventuellement des additifs choisis et/ou une atmosphère inerte à l'égard de la surface de l'implant, de préférence constituée d'azote, d'oxygène et/ou de gaz noble.

2. Implant selon la revendication 1, **caractérisé en ce que** celui-ci est constitué d'un alliage titane/zirconium qui contient éventuellement en outre du niobium, du tantale ou d'autres additifs métalliques compatibles avec les tissus.

3. Implant selon la revendication 1 et 2, **caractérisé en ce que** la surface hydroxylée et hydrophile a été obtenue par un procédé de décapage électrolytique ou chimique, de préférence par traitement avec un acide inorganique ou un mélange d'acides inorganiques, de préférence avec de l'acide fluorhydrique, de l'acide chlorhydrique, de l'acide sulfurique, de l'acide nitrique ou un mélange de ces acides ou de l'acide chlorhydrique, du peroxyde d'hydrogène et de l'eau dans un rapport pondéral d'environ 1:1:5.

4. Implant selon l'une des revendications 1 à 3, **caractérisé en ce que** l'intérieur de l'enveloppe est rempli au moins partiellement avec de l'eau pure contenant éventuellement d'autres additifs et **en ce que** la quantité d'eau présente est au moins telle que le mouillage de la surface rendue rugueuse de l'implant soit garanti, le volume résiduel dans l'enveloppe étant éventuellement rempli avec des gaz inertes à l'égard de la surface de l'implant, de préférence avec de l'oxygène, de l'azote des gaz nobles ou un mélange de ces gaz.

5. Implant selon l'une des revendications 1 à 4, **caractérisé en ce que** l'eau à l'intérieur de l'enveloppe présente une résistance électrique d'au moins 2 Mohm-cm et une teneur totale en carbone organique d'au plus 10 ppb.

6. Implant selon l'une des revendications 1 à 5, **caractérisé en ce que** l'eau à l'intérieur de l'enveloppe contient des cations alcalins monovalents et/ou des cations divalents sous forme de sels inorganiques solubles dans l'eau.

7. Implant selon l'une des revendications 1 à 6, **caractérisé en ce que** l'eau à l'intérieur de l'enveloppe contient comme cations alcalins monovalents Na⁺ ou K⁺ ou un mélange de Na⁺ et de K⁺, avec des anions correspondants sous forme de sels inorganiques, de préférence sous forme de chlorure de sodium, de chlorure de potassium, de chlorate de sodium ou de chlorate de potassium, de nitrate de sodium ou de nitrate de potassium, de phosphate de sodium ou de phosphate de potassium, de phosphonate de sodium ou de phosphonate de potassium ou d'un mélange de ces sels.

8. Implant selon l'une des revendications 1 à 7, **caractérisé en ce que** l'eau à l'intérieur de l'enveloppe contient comme cations divalents Mg⁺², Ca⁺², Sr⁺² ou Mn⁺² ou leurs mélanges, de préférence Mg⁺² et/ou Ca⁺², sous forme des chlorures.

9. Implant selon l'une des revendications 1 à 8, **caractérisé en ce que** l'eau à l'intérieur de l'enveloppe contient des cations et des anions, qui sont déjà présents dans le liquide corporel, de préférence dans chaque cas à la concentration physiologique.

10. Implant selon l'une des revendications 1 à 9, **caractérisé en ce que** l'eau à l'intérieur de l'enveloppe présente un indice d'acide dans la plage de 4 à 9, de préférence de 6 à 8.

11. Implant selon l'une des revendications 1 à 10, **caractérisé en ce que** la quantité totale des cations ou des anions à l'intérieur de l'enveloppe se situe dans chaque cas dans-la plage d'environ 50 méq/l à 250 méq/l, de préférence dans chaque cas dans la plage d'environ 100 méq/l à 200 méq/l et de préférence est dans chaque cas d'environ 150 méq/l et la quantité totale des cations divalents présents est de préférence de 1 méq/l à 20 méq/l.

12. Implant selon l'une des revendications 1 à 11, **caractérisé en ce que** la surface rendue rugueuse de l'implant est au moins 1,5 fois, de préférence 2 à 12 fois, de préférence au moins 2,5 à 6 fois, plus grande que la surface plane et polie correspondante.

13. Implant selon l'une des revendications 1 à 12, **caractérisé en ce que** l'angle de contact "supérieur" de la surface hydrophile de l'implant est inférieur à 50° et l'angle de contact "inférieur" est inférieur à 20°.

14. Implant selon l'une des revendications 1 à 13, **caractérisé en ce que** la surface hydroxylée et hydrophile de l'implant contient au plus 20 % atomiques de carbone.

15. Implant selon l'une des revendications 1 à 14, **caractérisé en ce que** l'enveloppe imperméable aux gaz et aux liquides constitue une ampoule soudée en verre, en métal, en polymère synthétique ou en un autre matériau imperméable aux gaz et aux liquides ou en une combinaison de ces matériaux, et **en ce que** le métal se présente de préférence sous forme d'une feuille métallique mince.

16. Procédé pour la fabrication d'un implant selon l'une des revendications 1 à 15, dans lequel on rend rugueuse la surface de l'implant par grenaillage, sablage et/ou utilisation d'une technique au plasma, puis
(i) on traite la surface rendue rugueuse mécaniquement ou par une technique au plasma, par un procédé de décapage électrolytique ou chimique jusqu'à la formation d'une surface hydroxylée et hydrophile, de préférence avec un acide inorganique ou un mélange d'acides inorganiques, de préférence avec de l'acide fluorhydrique, de l'acide chlorhydrique, de l'acide sulfurique, de l'acide nitrique ou un mélange de ces acides, ou de l'acide chlorhydrique, du peroxyde d'hydrogène et de l'eau dans un rapport pondéral d'environ 1:1:5,
(ii) on lave la surface avec de l'eau pure qui contient éventuellement des additifs, dans une atmosphère inerte à l'égard de la surface de l'implant, et
(iii) on conserve la surface, en renonçant à un autre traitement, dans une atmosphère inerte à l'égard de la surface de l'implant et/ou en permanence en présence d'eau pure qui contient éventuellement des additifs, de préférence dans une enveloppe qui est pratiquement imperméable aux gaz et aux liquides.
